# EUROPEAN PATENT APPLICATION

(11) **EP 4 803 068 A1**
(43) Date of publication of application: **09.09.2026**
(21) Application number: 26156327.4
(22) Date of filing: 04.02.2026
(51) Int. Cl.: A61K 8/06, A61K 8/27, A61K 8/29, A61K 8/46, A61K 8/49, A61K 8/89, A61K 8/90, A61Q 17/04

(54) **COSMETIC COMPOSITION FOR BLOCKING ULTRAVIOLET RAYS WITH EXCELLENT BLOCKING EFFECT AGAINST A WIDE RANGE OF UV RAYS AND COSMETIC CUSHION CONTAINING THE SAME**

(30) Priority: 27.02.2025 KR 20250026210
(71) Applicant: Kolmar Korea Co., Ltd., Sejong 30004 (KR)
(72) Inventor: YOO, Cha Young, 06800 Seoul (KR); JI, Yoon Sook, 06800 Seoul (KR); KIM, Yong Woo, 06800 Seoul (KR)
(74) Representative: Nederlandsch Octrooibureau

(57) **Abstract**

The present disclosure relates to a cosmetic composition for blocking ultraviolet rays (UV) with excellent blocking efficacy against a wide range of UV, and more particularly, to a cosmetic composition for blocking UV with excellent blocking efficacy against a wide range of UV, in which the potency of the UV-blocking agent is maintained over time even when impregnated into a cushion by optimally blending an organic UV-blocking agent, an inorganic UV-blocking agent, and an aqueous thickener, thereby exhibiting excellent UV-blocking efficacy, and in which various types of UV-blocking agents are stabilized within the formulation.

## Description

### CROSS-REFERENCE TO RELATED APPLICATION

This application claims priority to and the benefit of Korean Patent Application No. 10-2025-0026210, filed on Feb 27, 2025, the disclosure of which is incorporated herein by reference in its entirety.

### BACKGROUND

### 1. Field of the Invention

The present invention relates to a cosmetic composition for blocking ultraviolet rays (UV) with excellent blocking efficacy against a wide range of UV, and more particularly, to a cosmetic composition for blocking UV with excellent blocking efficacy against a wide range of UV, in which the potency of the UV-blocking agent is maintained over time even when impregnated into a cushion by optimally blending an organic UV-blocking agent, an inorganic UV-blocking agent, and an aqueous thickener, thereby exhibiting excellent UV-blocking efficacy, and in which various types of UV-blocking agents are stabilized within the formulation.

### 2. Discussion of Related Art

Ultraviolet rays (UV) reaching the earth's surface from the sun have short wavelengths and high energy, which may cause skin aging and wrinkles even with exposure for a short time. Ultraviolet A (UVA; wavelengths 320 to 400 nm), classified as long-wavelength UV, has a longer wavelength than other UV and may reach the dermis, causing tanning and long-term skin damage due to aging. Ultraviolet B (UVB; wavelengths 280 to 320 nm) directly affects the skin negatively, tanning and causing skin cancer. Ultraviolet C (UVC; wavelengths less than 280 nm), UV with the shortest wavelength, has the highest energy. Although UVC is completely absorbed by the ozone layer and only a small amount reaches the earth's surface, exposure to UVC is known to cause burns, skin cancer, and cataracts.

Therefore, countless sunscreen products are available to protect the skin from these UV rays.

UV-blocking products may lose their UV protection efficacy when exposed to sunlight outdoors or left on the skin for extended periods. Therefore, reapplying a UV-blocking product during daily activities is effective for protecting the skin from UV. However, a liquid UV-blocking product is inconvenient to apply outdoors, and women often experience makeup breakdowns during application, requiring touch-ups. To address these issues, products like sun cushions, which can deliver UV protection, are necessary.

However, because sunscreens are absorbed into the cushion over time, even prolonged storage may lead to a decrease in the product's UV protection efficacy.

The present inventors have continuously conducted research to address these issues and confirmed that it is possible to implement a cosmetic composition capable of stabilizing an oil-based organic UV-blocking agent, an aqueous organic UV-blocking agent, and an inorganic UV-blocking agent within an emulsified formulation, while not reducing the efficacy of the UV-blocking agent even when impregnated into a cushion such as a sponge and thus exhibiting a blocking effect against UV rays in a wide wavelength range for a long period of time, thereby completing the present invention.

### [Related Art Documents]

[Patent Document]
(Patent Document 1) KR 10-2022-0054447 A

### SUMMARY OF THE INVENTION

The present invention aims to provide a cosmetic composition for blocking ultraviolet (UV) rays that significantly improves the stability of various UV-blocking agents over time, thereby exhibiting excellent UV-blocking efficacy across a wide range of UV rays.

The technical problems of the present invention are not limited to those described above, and other technical problems that are not mentioned will be readily apparent to those skilled in the art from the descriptions below.

According to one embodiment of the present invention, there is provided a cosmetic composition for blocking ultraviolet rays (UV) in an emulsified formulation, including: an organic UV-blocking agent; an inorganic UV-blocking agent; and an aqueous thickener, wherein the organic UV-blocking agent includes at least one selected from the group consisting of polysilicone-15, methylene bis-benzotriazolyl tetramethylbutylphenol, and terephthalylidene dicamphor sulfonic acid.

In addition, the methylene bis-benzotriazolyl tetramethylbutylphenol may be included in an amount of 0.2% to 3% by weight based on a total weight of the cosmetic composition for blocking UV.

In addition, the terephthalylidene dicamphor sulfonic acid may be included in an amount of 0.5% to 6.5% by weight based on a total weight of the cosmetic composition for blocking UV.

In addition, the polysilicone-15 may be included in an amount of 0.01% to 10% by weight based on a total weight of the cosmetic composition for blocking UV.

In addition, the aqueous thickener may include an acrylate-based thickener.

In addition, the acrylate-based thickener may include polyacrylate crosspolymer-6.

In addition, the acrylate-based thickener may be included in an amount of 0.001% to 2% by weight based on a total weight of the cosmetic composition for blocking UV.

In addition, the aqueous thickener may further include poloxamer.

In addition, the poloxamer may be included in an amount of 0.01% to 5% by weight based on a total weight of the cosmetic composition for blocking UV.

In addition, the inorganic UV-blocking agent may include at least one of titanium dioxide and zinc oxide.

In addition, the UV-blocking cosmetic composition may be a water-in-oil (W/O) formulation or a water-in-silicone (W/S) formulation.

In addition, the cosmetic composition for blocking UV may be for impregnation into a cosmetic cushion.

In addition, the cosmetic composition for blocking UV may be an emulsified formulation of at least one of a cream, a lotion, a fluid, and a gel formulation.

According to another embodiment of the present invention, there is provided a cosmetic cushion impregnated with the cosmetic composition for blocking UV.

### DETAILED DESCRIPTION OF EXEMPLARY EMBODIMENTS

Unless defined otherwise, all technical and scientific terms used herein have the same meaning as commonly understood by one of ordinary skill in the art to which the invention pertains. Generally, the nomenclature used herein and the experiment methods which will be described later are those well known and commonly employed in the art. Furthermore, when describing embodiments of the present invention, when it is determined that detailed descriptions of related known configurations or functions would hinder understanding of the embodiments of the present invention, the detail descriptions will be omitted. Furthermore, while embodiments of the present invention will be described below, the technical spirit of the present invention is not limited or restricted thereto, and may be modified and implemented in various ways by those skilled in the art.

When a part is said to "comprise" a certain component, unless specified otherwise, this means that it may further include other components, rather than exclude other components. The term "and/or" as used herein encompasses a combination of a plurality of related items or any one of a plurality of related items.

According to an embodiment of the present invention, a cosmetic composition for blocking UV is provided in an emulsified form, which includes an aqueous phase and an oil phase, the cosmetic composition including: an organic UV-blocking agent; an inorganic UV-blocking agent; and an aqueous thickener, wherein the organic UV-blocking agent includes at least one selected from the group consisting of polysilicone-15, methylene bis-benzotriazolyl tetramethylbutylphenol, and terephthalylidene dicamphor sulfonic acid. The present inventors have confirmed that by including a specific aqueous thickener, the organic UV-blocking agent and the inorganic UV-blocking agent can be stabilized over time in the formulation, thereby maintaining the potency of the UV-blocking agent. In particular, when a composition including a UV-blocking agent is impregnated into a sponge or cushion, the potency of the organic UV-blocking agent tends to decrease significantly. However, the cosmetic composition for blocking UV of the present invention can maintain the potency of the UV-blocking agent over time even when impregnated into a sponge or cushion.

Polysilicone-15, a component having a structure represented by Chemical Formula 1 below, exhibits excellent UV-blocking efficacy in the wavelength range of 270 to 330 nm. The present inventors have confirmed that polysilicone-15 is not readily adsorbed into a sponge or cushion. Therefore, even when a cosmetic composition for blocking UV including polysilicone-15 is impregnated into a sponge or cushion, the potency of polysilicone-15 can be maintained similar to that immediately after preparation.

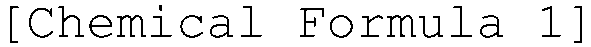

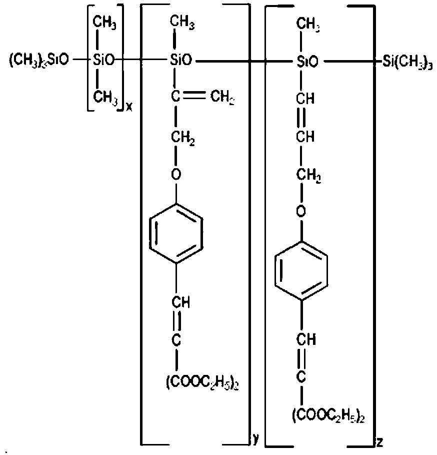

In one embodiment, polysilicone-15 may be included in an amount of 0.01% to 10% by weight, specifically 0.05% to 5% by weight, and more specifically 0.1% to 2% by weight, based on a total weight of the cosmetic composition for blocking UV. When polysilicone-15 is included in the above-described amount, the potency of the polysilicone-15 may be maintained over time, and the UV-blocking efficacy may be excellent. However, the present invention is not limited thereto.

In one embodiment, polysilicone-15 may be applied to an oil phase of the cosmetic composition for blocking UV of the present invention. Polysilicone-15, as an oil-soluble UV-blocking agent component, may exhibit excellent UV-blocking efficacy when applied to the oil phase.

The present inventors have confirmed that an optimal combination of an aqueous thickener can also improve the temporal stability of methylene bis-benzotriazolyl tetramethylbutylphenol, and terephthalylidene dicamphor sulfonic acid.

Methylene bis-benzotriazolyl tetramethylbutylphenol, a component having a structure represented by Chemical Formula 2 below, exhibits excellent UV-blocking efficacy in the wavelength range of 300 to 360 nm. The methylene bis-benzotriazolyl tetramethylbutylphenol is stable over time within the cosmetic composition for blocking UV of the present invention, so that even when the cosmetic composition for blocking UV of the present invention is impregnated into a sponge or cushion, the potency of the methylene bis-benzotriazolyl tetramethylbutylphenol can be maintained similar to that immediately after preparation.

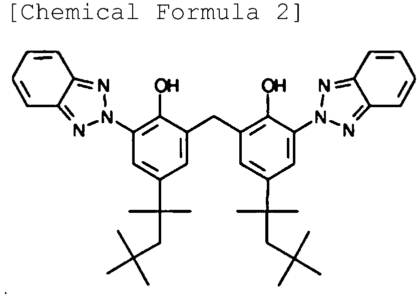

In one embodiment, methylene bis-benzotriazolyl tetramethylbutylphenol may be included in an amount of 0.2% to 3% by weight, specifically 0.3% to 3% by weight, based on a total weight of the cosmetic composition for blocking UV. When methylene bis-benzotriazolyl tetramethylbutylphenol is included in the above-described amount, the formulation can be stable over time and exhibit excellent UV protection efficacy. However, the present invention is not limited thereto.

In one embodiment, methylene bis-benzotriazolyl tetramethylbutylphenol may be applied to an aqueous phase of the cosmetic composition for blocking UV of the present invention. Methylene bis-benzotriazolyl tetramethylbutylphenol is a water-dispersible UV-blocking agent component that may be applied to the aqueous phase to exhibit excellent UV protection efficacy.

Terephthalylidene dicamphor sulfonic acid is a component having a structure represented by the following Chemical Formula 3 and exhibits excellent UV protection efficacy in the wavelength range of 320 to 400 nm. The terephthalylidene dicamphor sulfonic acid is stable over time within the cosmetic composition for blocking UV of the present invention, so that even when the cosmetic composition for blocking UV of the present invention is impregnated into a sponge or cushion, the potency of the terephthalylidene dicamphor sulfonic acid can be maintained similar to that immediately after preparation.

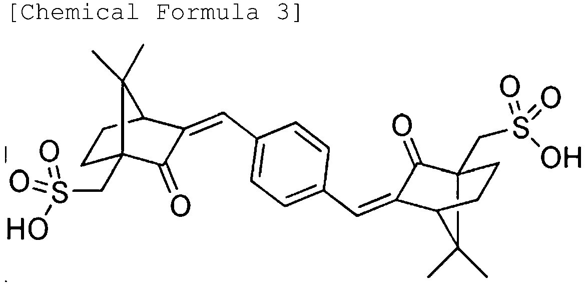

In one embodiment, terephthalylidene dicamphor sulfonic acid may be included in an amount of 0.5% to 6.5% by weight, specifically 0.7% to 6% by weight, and more specifically 1% to 5.5% by weight, based on a total weight of the cosmetic composition for blocking UV. When terephthalylidene dicamphor sulfonic acid is included in the above-described amount, the formulation may be stable over time and exhibit excellent UV-blocking efficacy. However, the present invention is not limited thereto.

In one embodiment, terephthalylidene dicamphor sulfonic acid may be applied to an aqueous phase of the cosmetic composition for blocking UV of the present invention. The terephthalylidene dicamphor sulfonic acid, as a water-soluble UV-blocking agent component, may be applied to the aqueous phase to exhibit excellent UV-blocking efficacy.

In one embodiment, the cosmetic composition for blocking UV of the emulsified formulation of the present invention can exhibit significantly excellent UV-blocking efficacy by including both methylene bis-benzotriazolyl tetramethylbutylphenol, and terephthalylidene dicamphor sulfonic acid in the water phase.

The inorganic UV-blocking agent of the present invention may exhibit excellent blocking efficacy against a wide range of ultraviolet rays in the wavelength range of 250 nm to 400 nm.

In one embodiment, the inorganic UV-blocking agent may include at least one of titanium dioxide and zinc oxide.

In one embodiment, the inorganic UV-blocking agent may be included in an amount of 0.1% to 40% by weight, specifically 0.5% to 35% by weight, and more specifically 1% to 30% by weight, based on a total weight of the cosmetic composition for blocking UV.

In one embodiment, at least one of polysilicone-15 and an inorganic UV-blocking agent may be applied to an oil phase, and at least one of methylene bis-benzotriazolyl tetramethylbutylphenol, terephthalylidene dicamphor sulfonic acid, and an inorganic UV-blocking agent may be applied to a water phase.

In one embodiment, the aqueous thickener may include an acrylate-based thickener. The present inventors have confirmed that when an acrylate-based thickener is included, the stability of the emulsion formulation is improved and the potency of the UV-blocking agent is enhanced.

In one embodiment, the acrylate-based thickener may include at least one of polyacrylate crosspolymer-6, carbomer, and ammonium acryloyldimethyltaurate/ vinylpyrrolidone (VP) copolymer, specifically polyacrylate crosspolymer-6.

In one embodiment, the acrylate-based thickener may be included in an amount of 0.001% to 2% by weight, specifically 0.005% to 1.5% by weight, and more specifically 0.01% to 1% by weight, based on the total weight of the cosmetic composition for blocking UV. When an acrylate-based thickener is included in this amount, the temporal stability of various organic UV-blocking agents can be appropriately enhanced. However, the present invention is not limited thereto.

In one embodiment, the aqueous thickener may further include poloxamer. It has been confirmed that the combination of the acrylate-based thickener and poloxamer can enhance the formulation stability of a cosmetic composition for blocking UV, and significantly improve usability and reduce stickiness.

In one embodiment, the poloxamer may include at least one selected from the group consisting of poloxamer 105, poloxamer 124, poloxamer 181, poloxamer 182, poloxamer 184, poloxamer 185, poloxamer 188, poloxamer 333, poloxamer 403, poloxamer 407, poloxamer 331, poloxamer 338, poloxamer 235, poloxamer 234, poloxamer 217, poloxamer 401, poloxamer 215, poloxamer 101, poloxamer 123, poloxamer 237, poloxamer 238, poloxamer 334, poloxamer 335, poloxamer 108, poloxamer 122, poloxamer 183, poloxamer 212, poloxamer 231, poloxamer 282, poloxamer 284, poloxamer 288, and poloxamer 402, and specifically, poloxamer 188.

In one embodiment, the poloxamer may be included in an amount of 0.01% to 5% by weight, specifically 0.05% to 3% by weight, and more specifically 0.1% to 2% by weight, based on a total weight of the cosmetic composition for blocking UV. When poloxamer is included in the above-described amount, the temporal stability of various organic UV-blocking agents can be appropriately enhanced. However, the present invention is not limited thereto.

In one embodiment, the cosmetic composition for blocking UV of the present invention may further include an oily thickener. By applying the oily thickener, the formulation stability can be further improved. In one specific embodiment, the oily thickener may be included without limitation as long as it corresponds to an ingredient commonly used in cosmetic compositions. Examples of the thickener may include at least one selected from the group consisting of dextrin behenate, dextrin laurate, dextrin palmitate, dextrin myristate, dextrin palmitate/ethylhexanoate, dextrin palmitate/hexyldecanoate, dextrin palmitate/stearate, stearoyl inulin, sucrose laurate, microcrystalline cellulose, polyamide-3, polyamide-8, hexamethylene diisocyanate (HDI)/trimethylol hexyllactone crosspolymer, VP/eicosene copolymer, glyceryl tribehenate/isostearate/eicosandioate, and glyceryl ethylhexanoate/stearate/adipate, and specifically, dextrin palmitate may be included. However, the present invention is not limited thereto.

In one embodiment, the oily thickener may be included in an amount of 0.01% to 10% by weight, specifically 0.05% to 5% by weight, more specifically 0.1% to 3% by weight, and most specifically 0.1% to 1% by weight, based on a total weight of the cosmetic composition for blocking UV. When the oily thickener is included in the above-described amount, the temporal stability of various organic UV-blocking agents can be appropriately improved. However, the present invention is not limited thereto.

In one embodiment, the cosmetic composition for blocking UV of the present invention may further include an emollient. The emollient serves as a solvent for an oil phase, maintains moisture in the skin, and provides a lasting moisturizing feeling. Examples of the emollient include silicone-based oils, hydrocarbon-based oils, and ester-based oils. However, the present invention is not limited thereto, and any oil commonly used in the art may be appropriately selected without limitation. In one specific embodiment, the emollient may include at least one ester-based oil of C12-15 alkyl benzoate, dibutyl adipate, butylene glycol dicaprylate/dicaprate, ethylhexyl stearate, and propylheptyl caprylate; at least one silicone-based oil of disiloxane, caprylyl methicone, and methyl trimethicone; and at least one hydrocarbon-based oil of isododecane, hydrogenated poly(C6-14 olefin), and C15-19 alkane, and specifically, at least one of butylene glycol dicaprylate/dicaprate and caprylyl methicone may be included.

In one embodiment, the cosmetic composition for blocking UV of the present invention may further include an emulsifier. The emulsifier may function to stably emulsify oil-phase components and water-phase components within the formulation. The emulsifier may include any emulsifier component commonly used in the art, and specifically, at least one of a water-in-oil emulsifier and a water-in-silicone emulsifier may be included. In a specific embodiment, the emulsifier may include at least one of PEG-10 dimethicone, cetyl polyethylene glycol (PEG)/polypropylene glycol (PPG)-10/1 dimethicone, polyglyceryl-4 diisostearate/polyhydroxystearate/sebacate, cetyl PEG/PPG-10/1 dimethicone*pentaerythrityl tetra-dit-butyl hydroxyhydrocinnamate, diisostearoyl polyglyceryl-3 dimer dilinoleate, lauryl polyglyceryl-3 polydimethylsiloxyethyl dimethicone, lauryl polyglyceryl-3 polydimethylsiloxyethyl dimethicone*tocopherol, sorbitan sesquioleate, and polyglyceryl-2 dipolyhydroxystearate, and specifically PEG-10 dimethicone may be included.

In one embodiment, the cosmetic composition for blocking UV of the present invention may further include a co-emulsifier. The co-emulsifier may contribute to the stabilization of an emulsified formulation formed by an oil-phase component and a water-phase component within the formulation. The co-emulsifier may include any co-emulsifier commonly used in the art, and in one specific embodiment, the co-emulsifier may include at least one of polyglyceryl-3 polydimethylsiloxyethyl dimethicone and polyglyceryl-6 polyricinoleate.

In one embodiment, the cosmetic composition for blocking UV of the present invention may further include an inorganic thickener. The inorganic thickener may be included in an oil phase to adjust the viscosity of the oil phase, thereby improving the long-term stability and usability of the formulation. The inorganic thickener may be included without limitation as long as it is an ingredient that may be commonly used in a cosmetic composition. In one specific embodiment, the inorganic thickener may include at least one selected from the group consisting of disteardimonium hectorite, hectorite, quaternium-18 hectorite, stearalkonium hectorite, dihydrogenated tallow benzylmonium hectorite, bentonite, quaternium-90 bentonite, quaternium-18 bentonite, and stearalkonium bentonite, and specifically, disteardimonium hectorite may be included.

In one embodiment, the cosmetic composition for blocking UV of the present invention may further include a moisturizer, a preservative, or a functional ingredient.

The moisturizer provides moisture to the skin, maintains moisture in the skin, and provides a lasting moisturizing sensation. Preferably, the moisturizer is a water-soluble ingredient. For example, at least one of glycerin, butylene glycol, propylene glycol, dipropylene glycol, methylpropanediol, pentylene glycol, glycereth-26, propanediol, sorbitol, and diglycerin may be used, but the present invention is not limited thereto.

The preservative is used to enhance the preservation effect. For example, at least one of ethylhexylglycerin, 1,2-hexanediol, caprylyl glycol, phenoxyethanol, and glyceryl caprylate may be used, but the present invention is not limited thereto. Any preservative commonly used in the art may be appropriately selected without limitation.

As the functional ingredient, various ingredients may be mixed and added in various combinations depending on the purpose of the cosmetic composition. Examples include wrinkle-improving agents, skin-brightening functional ingredients, pigmentation-reducing functional ingredients, skin-soothing functional ingredients, and sebum- or pore-managing functional ingredients.

In addition, the cosmetic composition for blocking UV of the present invention may further include ingredients commonly included in cosmetic compositions. For example, dispersants, polyols, usability-modifying agents, skin conditioning agents, ion sequestrants, bactericides, pH regulators, antioxidants, alcohols, plant extracts, fragrances, and the like may be included.

The blending amounts of the above-described ingredients are not particularly limited and may be easily selected by those skilled in the art within a range that does not impair the purpose and effects of the present invention.

In one embodiment, the cosmetic composition for blocking UV of the present invention may be formulated as an emulsion, specifically, an emulsion in which an aqueous phase is a dispersed phase. The emulsion in which the aqueous phase is the dispersed phase may be a water-in-oil (W/O) formulation or a water-in-silicone (W/S) formulation.

Furthermore, in addition to the water-in-oil or water-in-silicone formulations, it may also be formulated as a multiple emulsion, such as an oil-in-water-in-oil (O/W/O) formulation.

In addition, the cosmetic composition for blocking UV of the present invention may be provided in a formulation commonly manufactured in the art. For example, it may be formulated as a solubilized formulation. However, the composition is not limited thereto, and may also be provided in various formulations, such as a solution, a suspension, an emulsion, a solid, an oil, powder, a paste, or a foam, an aerosol, or mist.

In one embodiment, the cosmetic composition for blocking UV of the present invention may be provided in a gel formulation, a lotion formulation, a cream formulation, a low-viscosity liquid formulation, or a fluid formulation. Specifically, the cosmetic composition may be provided in a lotion formulation, and more specifically, the formulations may correspond to emulsified formulations. However, the present invention is not limited thereto.

In one embodiment, the cosmetic composition for blocking UV of the present invention may be used as a cosmetic composition for a cosmetic cushion.

According to another embodiment of the present invention, a cosmetic cushion impregnated with the cosmetic composition for blocking UV of the present invention is provided. The cosmetic composition exhibits excellent stability of the UV-blocking agent over time, thereby resolving the problem that the potency of the UV-blocking agent component is significantly reduced when the cosmetic composition for blocking UV is impregnated into a cushion or sponge.

In one embodiment, the cosmetic cushion may be made of various materials known in the art. In one specific embodiment, the cosmetic cushion may be a polyurethane sponge. However, the present invention is not limited thereto.

In one embodiment, the cosmetic cushion may be commercialized as a sun cushion product with excellent UV-blocking efficiency.

In one embodiment, the cosmetic composition for blocking UV of the present invention may be manufactured into various cosmetics with excellent UV-blocking efficiency, in addition to the sun cushion product. Examples of the cosmetics include sun care products such as sunscreens, sun sticks, sun milks, sun sprays, and sun lotions; functional cosmetics such as nourishing creams, essences, ampoules, moisturizing creams, and eye creams; and basic cosmetics such as toners and lotions. However, the present invention is not limited thereto.

Hereinafter, the following examples and experimental examples are presented to further illustrate the present invention, but the present invention is not limited thereto.

### Experimental Example 1. Confirmation of temporal stability of polysilicone-15 - Potency analysis

To identify an organic UV-blocking agent suitable for sponges and cushions, plastic vials or polyurethane sponges were impregnated with organic UV-blocking agents, and their temporal stability was confirmed through potency analysis. Specifically, polysilicone-15, ethylhexyl salicylate, ethylhexyl triazone, diethylamino hydroxybenzoyl hexyl benzoate, and bis-ethylhexyloxyphenol methoxyphenyl triazine were each used as organic UV-blocking agents. Each of these organic UV-blocking agents was dissolved in a butylene glycol dicaprylate/dicaprate solvent at a concentration of 3% by weight and impregnated into plastic vials or polyurethane sponges. The potency of the plastic vials and polyurethane sponges containing the organic UV-blocking agent was measured, the samples were stored for one month under harsh conditions (40 °C) or room temperature (25 °C). The potency of the stored samples was then remeasured to confirm the stability of the organic UV-blocking agent over time.

Table 1 shows the results of the potency analysis using the above-described method.

**[Table 1]**

| **Test Item (UV Filter)** | **Sample type** | **Labeled amount of organic UV-blocking agent (% by weight)** | **Potency - Content relative to Labeled amount (%)** | | |
|---|---|---|---|---|---|
| | | | **Initial** | **Accelerated** | **Room Temp.** |
| **Polysilicone-15** | Vial | 3 | 101.6 | 100.8 | 101.2 |
| | Impregnated | 3 | 102.0 | 101.6 | 101.8 |
| **Ethylhexyl salicylate** | Vial | 3 | 100.2 | 99.9 | 100.6 |
| | Impregnated | 3 | 98.0 | 94.2 | 95.5 |
| **Ethylhexyl triazone** | Vial | 3 | 99.8 | 98.7 | 99.0 |
| | Impregnated | 3 | 98.6 | 89.2 | 92.4 |
| **Diethylamino hydroxybenzoyl hexyl benzoate** | Vial | 3 | 98.9 | 96.6 | 97.5 |
| | Impregnated | 3 | 97.6 | 91.9 | 93.2 |
| **Bis-ethylhexyloxyphenol methoxyphenyl triazine** | Vial | 3 | 103.2 | 102.5 | 103.2 |
| | Impregnated | 3 | 101.4 | 91.2 | 94.8 |

According to Table 1 above, the organic UV-blocking agents, except for polysilicone-15, showed a tendency for their potency to decrease over time under both harsh conditions and room temperature conditions when impregnated into a sponge and stored, compared to when stored in a vial, and thus were confirmed to be unsuitable UV-blocking agent components for application to a sponge. On the contrary, polysilicone-15 was confirmed to maintain its potency similar to the initial value in both the vial and the polyurethane sponge, and thus was confirmed to be a suitable UV-blocking agent component for impregnation into a sponge.

### Experimental Example 2. Analysis of UV-blocking agent potency by component

### (1) Preparation of Example 1

Example 1, a water-in-oil formulation, was prepared as shown in Table 2 below. Specifically, as organic UV-blocking agents, polysilicone-15, which was a water-soluble organic UV-blocking agent that was confirmed to have excellent stability over time even when impregnated into a sponge through the above Experimental Example 1; terephthalylidene dicamphor sulfonic acid (TDSA)*water (TDSA content: 33%), a water-dispersible organic UV-blocking agent; and methylene bis-benzotriazolyl tetramethylbutylphenol (MBBT)*decyl glucoside*glycerin*xanthan gum*water (MBBT content: 50%), were used. As inorganic UV-blocking agents, titanium dioxide*C12-15 alkyl benzoate*polyhydroxystearic acid*stearic acid*aluminum hydroxide (titanium dioxide content: 44%) and zinc oxide*dibutyl adipate*polyhydroxystearic acid*stearic acid (zinc oxide content: 72%) were used. Polyacrylate crosspolymer-6 and poloxamer 188 were used as aqueous thickeners, and dextrin palmitate was used as oily thickener. The unit for the contents in Table 2 is % by weight

**[Table 2]**

| **Phase** | **Role** | **Component** | **Content (% by weight)** |
|---|---|---|---|
| **Oil Phase** | **Organic UV-blocking agent** | Polysilicone-15 | 1 |
| | **Inorganic UV-blocking agent** | Titanium dioxide*C12-15 alkyl benzoate*polyhydroxystearic acid*stearic acid*aluminum hydroxide | 18 |
| | | Zinc oxide*dibutyl adipate*polyhydroxystearic acid*stearic acid | 8 |
| | **Emollient** | Butylene glycol dicaprylate/dicaprate | 8 |
| | | Caprylyl methicone | 7 |
| | **Oily thickener** | Dextrin palmitate | 0.2 |
| | **Inorganic Thickener** | C13-15 alkane*polyglyceryl-3 polyricinoleate*disteardimonium hectorite | 5 |
| | **Emulsifier** | PEG-10 dimethicone | 1.5 |
| | **Co-emulsifier** | Polyglyceryl-3 polydimethylsiloxyethyl dimethicone | 0.5 |
| | | Polyglyceryl-6 polyricinoleate | 1 |
| | **Powder** | Methyl methacrylate crosspolymer | 2 |
| | **Preservative** | Caprylyl glycol | 0.1 |
| | | Glyceryl caprylate | 0.1 |
| | | Ethylhexylglycerin | 0.1 |
| **Water phase** | **Organic UV-blocking agent** | Methylene bis-benzotriazolyl tetramethylbutylphenol*decyl glucoside*glycerin*xanthan gum*water | 6 |
| | | Terephthalylidene dicamphor sulfonic acid*water | 4 |
| | **Aqueous thickener** | Polyacrylate crosspolymer-6 | 0.02 |
| | | Poloxamer 188 | 0.2 |
| | **Dispersant** | Sodium stearoyl glutamate | 0.08 |
| | **Skin Conditioning Agent** | Niacinamide | 2 |
| | | Adenosine | 0.04 |
| | **Moisturizer** | Propanediol | 5 |
| | | Glycerin | 1 |
| | | 1,2-Hexanediol | 0.5 |
| | **Neutralizer** | Tromethamine | 0.6 |
| | **Solvent** | Purified Water | To 100 |

The specific preparation method is as follows.

The oil phase was heated to 70 to 80 °C to dissolve and disperse, and the water phase was heated to 70 to 80 °C to ensure sufficient dispersion. Next, the uniformly dispersed oil and water phases were cooled to 40 to 70 °C. The water phase was slowly added to the oil phase to emulsify, and then cooled to room temperature to complete the cosmetic composition for blocking UV of Example 1.

### (2) Potency analysis by UV-blocking agent

The cosmetic composition for blocking UV of Example 1 was impregnated into a sponge (finished product), and the potency was measured. The composition was then stored for 1 month and 3 months under the same temperature conditions as Experimental Example 1. The potency was measured after each period to evaluate the stability of the UV-blocking agent over time. In addition, to confirm whether the UV-blocking agent within the composition of Example 1 was stabilized, the composition of Example 1 was placed in a vial (semi-finished product) without impregnating the sponge. The composition was then stored for 1 month and 3 months under the same temperature conditions as Experimental Example 1, and the potency was measured after each period. The potency measurements based on the evaluation results are shown in Table 3. Except for polysilicone-15, the amounts of UV-blocking agents were labeled based on the proportion of UV-blocking agents within the raw materials used.

**[Table 3]**

| **Component** | **Sample type** | **Labeled amount (% by weight)** | **Potency - Content relative to labeled amount (%)** | | | | |
|---|---|---|---|---|---|---|---|
| | | | Initial | Accelerated (1 month) | Room Temp. (1 month) | Accelerated (3 months) | Room Temp. (3 months) |
| **Polysilicone-15** | Semi-finished product | 1 | 97.3 | 104.6 | 106.5 | 102.4 | 101.9 |
| | Finished product | | 99.3 | 107.0 | 105.3 | 109.9 | 106.8 |
| **Titanium Dioxide** | Semi-finished product | 7.92 | 100.4 | 101.8 | 99.7 | 101.5 | 101.1 |
| | Finished product | | 105.2 | 103.9 | 103.7 | 107.7 | 103.2 |
| **Zinc Oxide** | Semi-finished product | 5.76 | 96.9 | 104.1 | 101.9 | 104.7 | 104.5 |
| | Finished product | | 100.2 | 105.2 | 105.2 | 110.2 | 105.2 |
| **Methylene bis-benzotriazolyl tetramethylbutylphenol** | Semi-finished product | 2 | 102.2 | 99.6 | 100.2 | 98.6 | 100.0 |
| | Finished product | | 104.6 | 103.9 | 101.3 | 102.4 | 101.5 |
| **Terephthalylidene dicamphor sulfonic acid** | Semi-finished product | 1.98 | 98.8 | 97.8 | 97.5 | 99.4 | 97.9 |
| | Finished product | | 107.7 | 103.1 | 101.1 | 105.8 | 102.3 |

According to Table 3 above, both the organic and inorganic UV-blocking agents included in Example 1 had similar potency immediately after impregnation into the sponge, demonstrating excellent stability over time.

Therefore, it was confirmed that the organic and inorganic UV-blocking agents of the present invention are stabilized within the cosmetic composition, and even when impregnated into a cosmetic cushion, the UV-blocking agent's potency remains stable, thereby demonstrating excellent UV-blocking efficacy.

### Experimental Example 3. Comparative experiment according to UV-blocking agent content

### (1) Preparation of examples and comparative examples

As shown in Tables 4 and 5 below, based on Example 1, only the content of terephthalylidene dicamphor sulfonic acid*water or methylene bis-benzotriazolyl tetramethylbutylphenol*decyl glucoside*glycerin*xanthan gum*water applied to the aqueous phase among the organic UV-blocking agents was varied, and the remaining components and contents were kept the same to preparing Examples and Comparative Examples.

**[Table 4]**

| | **Example** | | | | | | | |
|---|---|---|---|---|---|---|---|---|
| | **2** | **3** | **4** | **5** | **6** | **7** | **8** | **9** |
| **Methylene bis-benzotriazolyl tetramethylbutylphenol*decyl glucoside*glycerin*xanthan gum*water** | 3 | 3 | 3 | 3 | 1 | 2 | 5 | 6 |
| **Terephthalylidene dicamphor sulfonic acid*water** | 2 | 4 | 8 | 10 | 6 | 6 | 6 | 6 |

**[Table 5]**

| | **Comparative Example** | | | | | |
|---|---|---|---|---|---|---|
| | **1** | **2** | **3** | **4** | **5** | **6** |
| **Methylene bis-benzotriazolyl tetramethylbutylphenol*decyl glucoside*glycerin*xanthan gum*water** | 3 | 3 | 3 | - | 0.5 | 7 |
| **Terephthalylidene dicamphor sulfonic acid*water** | 1 | 15 | 20 | 6 | 6 | 6 |

### (2) Long-term stability and sensory evaluation

The long-term stability of the examples and comparative examples was evaluated, as well as the degree of stickiness and white turbidity upon application.

For the long-term stability evaluation, the formulations were stored for one month at a low temperature (5 °C), room temperature (25 °C), and a high temperature (40 °C), and evaluated to determine whether they remained as stable as immediately after manufacturing. When the formulation was not stable at any temperature, it was evaluated as a failure.

For the degree of stickiness, the examples and comparative examples were applied to the skin, and the degree of stickiness and smooth application were evaluated using a 5-point scale, with lower scores assigned to lower stickiness. For the degree of white turbidity, after applying Examples or Comparative Examples to the skin, the degree of white turbidity was visually observed. Higher scores were assigned to improved white turbidity, using a 5-point scale.

The evaluation results obtained by the above-described method are shown in Tables 6 to 7 below.

### (3) Measurement of UV-blocking efficacy

To confirm the increased UV-blocking efficacy of Examples and Comparative Examples, the protection grade (PA) and sun protection factor (SPF) measurements were performed *in vitro.* Specifically, 2.0 mg/cm² of each of the examples and comparative examples were evenly applied to poly(methyl methacrylate) (PMMA) plates for 30 seconds and then dried in a darkroom for 15 minutes to prepare samples. UVAPF and *in vitro* SPF values were measured using a Labsphere UV 2000S *In Vitro* SPF analyzer. The UVAPF and *in vitro* SPF values are the averages of three experiments, and the results are shown in Tables 6 and 7 below.

**[Table 6]**

| | **Example** | | | | | | | | |
|---|---|---|---|---|---|---|---|---|---|
| | **1** | **2** | **3** | **4** | **5** | **6** | **7** | **8** | **9** |
| **Long-term stability** | ○ | ○ | ○ | ○ | ○ | ○ | ○ | ○ | ○ |
| **Stickiness** | 1 | 1 | 1 | 2 | 2 | 1 | 1 | 1 | 3 |
| **White turbidity** | 1 | 1 | 1 | 1 | 2 | 1 | 1 | 2 | 3 |
| **UVAPF** | High | Mid | High | High | High | High | High | High | High |
| ***in vitro* SPF** | 65 | 60 | 64 | 67 | 68 | 50 | 52 | 63 | 69 |

**[Table 7]**

| | **Comparative Example** | | | | | |
|---|---|---|---|---|---|---|
| | 1 | 2 | 3 | 4 | 5 | 6 |
| **Long-term stability** | ○ | X | X | ○ | ○ | X |
| **Stickiness** | 1 | 5 | 5 | 1 | 1 | 4 |
| **White turbidity** | 1 | 1 | 2 | 1 | 1 | 4 |
| **UVAPF** | Low | High | High | Low | Low | High |
| ***in vitro* SPF** | 49 | 68 | 69 | 32 | 40 | 54 |

According to Table 6, when appropriate amounts of MBBT and TDSA are included, a formulation with long-term stability is formed. In addition, the formulation is less sticky, making it convenient to use. Furthermore, the formulation exhibits minimal white turbidity so that a large amount can be easily applied. Furthermore, it was confirmed that the organic and inorganic UV-blocking agent components are well stabilized within the formulation, resulting in excellent UVAPF and SPF values and demonstrating significantly superior UV-blocking efficacy across a wide wavelength range.

On the other hand, when either MBBT or TDSA was included in an amount less than the optimal amount, the UV-blocking efficacy was significantly reduced. When the amount exceeds the optimal amount, the stickiness increased and the formulation stability deteriorated. In particular, high amounts of MBBT significantly worsen white turbidity, making the formulation unsuitable for use in cosmetic compositions.

Therefore, it was confirmed that appropriately adjusting the amount of MBBT and TDSA in the aqueous phase, the dispersed phase, is crucial for achieving a cosmetic composition with excellent UV-blocking efficacy across a wide wavelength range, formulation stability, and usability.

### Experimental Example 4. Comparative experiments according to type of aqueous thickener

### (1) Preparation of examples and comparative examples

As shown in Table 8 below, the examples and comparative examples were prepared based on Example 1, with only the components and/or contents of the aqueous thickener changed, while the remaining components and contents remained the same.

**[Table 8]**

| **Role** | **Component** | **Example** | | | | **Comparativ e Example** | |
|---|---|---|---|---|---|---|---|
| | | **1** | **10** | **11** | **12** | **7** | **8** |
| | Polyacrylate crosspolymer-6 | 0.0 2 | 0.0 2 | - | - | - | - |
| | Poloxamer 188 | 0.2 | - | - | - | - | - |
| **Aqueous thickener** | Carbomer | - | - | 0.0 2 | - | - | - |
| | Ammonium acryloyldimethyltaurate/VP copolymer | - | - | - | 0.0 2 | - | - |
| | Polyquaternium-39 | - | - | - | - | 0.02 | - |
| | Xanthan gum | - | - | - | - | - | 0.02 |

### (2) Long-term stability and sensory evaluation

The examples and comparative examples prepared as shown in Table 8 were evaluated for long-term stability and stickiness using the same method as Experimental Example 3. In addition, the application process was evaluated to determine whether the cosmetic compositions achieved an appropriate usability when applied, depending on the variation in the thickener.

The results of the evaluation using the above method are shown in Table 9.

**[Table 9]**

| | **Example 1** | **Example 10** | **Example 11** | **Example 12** | **Comparative Example 7** | **Comparative Example 8** |
|---|---|---|---|---|---|---|
| **Long-term stability** | ○ | ○ | ○ | ○ | x | x |
| **Stickiness** | 1 | 3 | 3 | 3 | 2 | 4 |
| **Usability** | Excellent | Excellent | Excellent | Excellent | Poor | Poor |

According to Table 9 above, it was confirmed that Examples 10 to 12 including an acrylate-based thickener as an aqueous thickener exhibited excellent formulation stability and satisfactory stickiness, and the application was easy, resulting in an excellent usability. In particular, Example 1 including both an acrylate-based thickener and a poloxamer was confirmed to have a significantly improved stickiness compared to Examples 10 to 12 and to exhibit the best usability. On the other hand, in the cases of Comparative Examples 7 and 8 including no acrylate-based thickener, Comparative Example 7, which included a cationic thickener, experienced a phase separation after high-temperature storage, and the formulation was confirmed to aggregate during the application process. Comparative Example 8, which included a polysaccharide-based thickener, experienced a phase separation after high-temperature storage, and the formulation was confirmed to be pushed during the application process, resulting in an inferior usability.

Therefore, it was confirmed that including an acrylate-based thickener as an aqueous thickener is an important factor in implementing a cosmetic composition for blocking UV with excellent usability, and that the stickiness can be significantly improved when poloxamer is additionally included.

The cosmetic composition for blocking UV of the present invention stabilizes various types of organic and inorganic UV-blocking agents within its formulation, thereby demonstrating excellent UV-blocking efficacy across a wide range of UV rays.

Furthermore, the cosmetic composition for blocking UV exhibits excellent stability over time, and therefore, even when impregnated into cushions or other products, the UV-blocking agent maintains its potency so that excellent UV-blocking efficacy can be exhibited for a long period of time. This makes it suitable for use in products such as sun cushions.

Furthermore, due to this stability over time, the composition is suitable for use in at least one emulsified formulation, including a cream, a lotion, a fluid, and a gel.

Furthermore, the cosmetic composition can significantly improve skin-brightening while containing a high concentration of inorganic UV-blocking agent, and can be easily used due to its low stickiness despite application of high concentrations of UV-blocking agents.

Furthermore, by including the UV-blocking agents in both the continuous and dispersed phases of the emulsified formulation, the cosmetic composition can reduce variations in UV-blocking efficacy depending on the size of the emulsified particles, thereby achieving uniform UV-blocking efficacy.

While specific aspects of the present invention have been described in detail above, it will be apparent to those skilled in the art that these specific descriptions merely represent preferred embodiments and are not intended to limit the scope of the present invention. Therefore, the substantial scope of the present invention is defined by the appended claims and their equivalents.

## Claims

1. A cosmetic composition for blocking ultraviolet rays (UV) in an emulsified formulation, comprising:
an organic UV-blocking agent;
an inorganic UV-blocking agent; and
an aqueous thickener,
wherein the organic UV-blocking agent includes at least one selected from the group consisting of polysilicone-15, methylene bis-benzotriazolyl tetramethylbutylphenol, and terephthalylidene dicamphor sulfonic acid.

2. The cosmetic composition for blocking UV of claim 1, wherein the methylene bis-benzotriazolyl tetramethylbutylphenol is included in an amount of 0.2% to 3% by weight based on a total weight of the cosmetic composition for blocking UV.

3. The cosmetic composition for blocking UV of claim 1, wherein the terephthalylidene dicamphor sulfonic acid is included in an amount of 0.5% to 6.5% by weight based on a total weight of the cosmetic composition for blocking UV.

4. The cosmetic composition for blocking UV of claim 1, wherein the polysilicone-15 is included in an amount of 0.01% to 10% by weight based on a total weight of the cosmetic composition for blocking UV.

5. The cosmetic composition for blocking UV of claim 1, wherein the aqueous thickener includes an acrylate-based thickener.

6. The cosmetic composition for blocking UV of claim 5, wherein the acrylate-based thickener is included in an amount of 0.001% to 2% by weight based on a total weight of the cosmetic composition for blocking UV.

7. The cosmetic composition for blocking UV of claim 5, wherein the acrylate-based thickener includes polyacrylate crosspolymer-6.

8. The cosmetic composition for blocking UV of claim 5, wherein the aqueous thickener further includes poloxamer.

9. The cosmetic composition for blocking UV of claim 8, wherein the poloxamer is included in an amount of 0.01% to 5% by weight based on a total weight of the cosmetic composition for blocking UV.

10. The cosmetic composition for blocking UV of claim 1, further comprising an oily thickener.

11. The cosmetic composition for blocking UV of claim 1, wherein the inorganic UV-blocking agent includes at least one of titanium dioxide and zinc oxide.

12. The cosmetic composition for blocking UV of claim 1, wherein the UV-blocking cosmetic composition is a water-in-oil (W/O) formulation or a water-in-silicone (W/S) formulation.

13. The cosmetic composition for blocking UV of claim 1, wherein the cosmetic composition for blocking UV is for impregnation into a cosmetic cushion.

14. The cosmetic composition for blocking UV of claim 1, wherein the cosmetic composition for blocking UV is an emulsified formulation of at least one of a cream, a lotion, a fluid, and a gel formulation.

15. A cosmetic cushion impregnated with the cosmetic composition for blocking UV of any one of claims 1 to 14.
